# EUROPEAN PATENT SPECIFICATION

(11) **EP 0 513 064 B1**
(45) Date of publication and mention of the grant of the patent: **18.12.1996**
(21) Application number: 91902872.0
(22) Date of filing: 29.01.1991
(51) Int. Cl.: B03C 5/00, C12Q 1/24, G01N 33/537, G01N 33/543

(54) **MANIPULATION OF SOLID, SEMI-SOLID OR LIQUID MATERIALS**
MANIPULATION VON FESTEN, SEMI-FESTEN ODER FLÜSSIGEN MATERIALIEN
MANIPULATION DE SUBSTANCES SOLIDES, SEMI-SOLIDES OU LIQUIDES

(30) Priority: 30.01.1990 GB 9002092
(43) Date of publication of application: 19.11.1992
(73) Proprietor: BRITISH TECHNOLOGY GROUP LIMITED, London SE1 6BU (GB)
(72) Inventor: PETHIG, Ronald, Anglesey, Gwynedd LL59 5DT (GB); BURT, Julian Paul Hillhouse, Bangor, Gwynedd LL57 4LN (GB)
(74) Representative: Buttrick, Richard
(86) International application number: GB9100122
(87) International publication number: WO9111262

(56) References cited:
- US-A- 4 390 403
- US-A- 4 441 972
- Conference Record of the 1986 IEEE Industry Applications Society Annual Meeting, Part II, 1986, R.J. Adamson et al.:"An automated stream centred dielectrophoretic system", see pages 1350-1354
- Biochimica et Biophysica Acta, volume 964, 1988, Elsevier, (Amsterdam, NL), J.
- A.R. Price et al.: "Applications of a new optical technique for measuring the dielectrophoretic behaviour of microorganisms", see pages 221-230
- Journal of Physics E. Scientific Instruments volume 22, November 1989, (London, GB), J.P.H. Burt et al.: "An optical dielectrophoresis spectrometer for low- frequency measurements on colloidal suspensions", see pages 952-957
- IEEE Transactions on Industry Applications, volume 24, no. 2, March/April 1988, (New York, US), S. Masuda et al.: "Movement of blood cells in liquid by nonuniform travelling field", see pages 217-222
- Biological Membranes, volume 5, 1984, W.M. Arnold et al.: "Electric field- induced fusion and rotation of cells", see pages 389-454

## Description

This invention relates to promoting reactions between particles suspended in a liquid.

A wide variety of commercial processes involve the use of liquid media having solid, semi-solid or liquid particles suspended in them. The particles may vary very widely from inert inorganic materials through to reactive materials, and organic or biological structures such as cells or parts of cells.

It has been known for some time that particles of these various types may be caused to move within a liquid medium by the use of a non-uniform electric field, and the basic phenomenon of dielectrophoresis has been extensively discussed, for example see "Dielectrophoresis", Cambridge University Press, 1978 by H A Pohl and Chapter 6 of "Dielectric and Electronic Properties of biological Materials", John Wiley & Sons 1979 by Ronald Pethig.

Recently the application of dielectrophoresis has been suggested in the area of materials classification: the construction of a so-called "optical dielectrophoresis spectrometer" is described in Burt, Al-Ameen & Pethig, "An optical dielectrophoresis spectrometer for low-frequency measurements on colloidal suspensions", Journal of Physics, section E, Scientific Instrumentation, Volume 22 (1989) pages 952 to 957.

That paper, and the related paper "Applications of a New Optical Technique for Measuring the Dielectrophoretic Behaviour of Microorganisms", Price, Burt and Pethig Biochimica et Biophysica Acta 964 (1988) pages 221 to 230, disclose the use of interdigitated electrodes deposited on a dielectric substrate to cause movement of suspended particulates by the dielectrophoretic effect.

Most previous work has been directed to the characterisation of materials by taking appropriate measurements of their electric field-induced properties. Another major application is the use of positive dielectrophoretic forces to align biological cells between electrodes prior to their electrofusion, as described by W M Arnold and U Zimmermann ("Electric Field Induced Fusion and Rotation of Cells", Biological Membranes 5, 389-454, 1984). Also, a method and apparatus for dielectrophoretic manipulation of chemical species has been described by J S Batchelder (US Patent 4,390,403, June 28, 1983). This method employs DC non-uniform electrical fields to manipulate one or more chemicals within a multi-electrode chamber so as to promote chemical reactions between the chemical species. The applied voltage may be periodically reversed in sign to decrease ionic shielding effects (see column 3, line 62 to column 4, line 3). The manipulation of the chemicals is controlled by positive dielectrophoretic forces resulting from differences in the dielectric constants of the chemical species.

In previous works of S Masuda, M Washizu and I Kawabata "Movement of Blood Cells in Liquid by Nonuniform Travelling Field", IEEE Transactions on Industry Applications, Volume 24 (1988) pages 217 to 222, blood cells were caused to move under the influence of a non-uniform travelling electric field. This field was generated by applying two, fixed frequency, multiphased, voltage signals, related by having the same frequency and amplitude, to a series of parallel electrodes. Likewise, the rotating electric field described by W M Arnold and U Zimmerman and employed to cause rotation of a single cell, is generated using either a single, phase-split, voltage signal or synchronised, identical, voltage pulses.

Summarising this background, in promoting reactions between particles suspended in a liquid, by using dielectrophoresis, the particles being of one type or more than one type, it is known to proceed by introducing a volume of the liquid with particles suspended therein into a treatment cell provided with electrodes, and applying an alternating electric signal of relatively high frequency to the electrodes to generate redistribution of the particles under the effect of dielectrophoretic force.

We have now found that if two or more non-uniform electric fields of differing frequencies are imposed, simultaneously or sequentially, on a suspension of particles of one or more than one type in a liquid, using appropriate electronic control, various reactions may be stimulated to occur in the particles in the liquid.

Thus, in accordance with a first feature of the invention, the method summarized above may be improved by applying, while the particles in the said cell occupy the redistributed positions, a second alternating electric signal to the electrodes, the frequency of the second signal being significantly different from that of the first signal, and chosen taking into account the dielectric constant and electrical conductivity of the particles and the liquid to produce dielectrophoretic forces acting on the particles differing from those produced by the first signal, and effecting a second redistribution of the particles differing from the first redistribution.

In accordance with an alternative feature of the invention, the method summarized above may be improved by simultaneously applying a second alternating electric signal to the electrodes, the frequency of the second signal being significantly different from that of the first signal and chosen taking into account the dielectric constant and electrical conductivity of the particles and the liquid to produce dielectrophoretic forces acting on the particles differing from those produced by the first signal.

The term reaction as used herein is to be interpreted broadly as covering various chemical, biochemical and physical interactions, and large scale manipulations such as separation followed by recombination, optionally with a treatment being selectively applied to one component of a multicomponent system while so separated from the other component(s).

Whereas previous work (e.g. Batchelder) has employed differences in the dielectric constant of the manipulated chemical species to control the desired reactions, we have found that, in addition to varying the dielectric constant, varying the electrical conductivity of either or both the suspended particles and the suspending medium provides a further degree of control. In this connection (and throughout this specification) the term dielectric constant is used to refer to the real part of the complex permittivity.

The particles which may be used may be of animate or inanimate material and they may be colloidal or of some other nature.

With appropriate choice of the dielectric constant and electrical conductivity of either or both the suspended particle and suspending medium, both positive and negative dielectrophoretic forces may be employed as the manipulating agent. Preferably, in carrying out the method of the invention, at least one of the electrical fields is chosen to effect a negative dielectrophoretic force on some only of the particles in the liquid. By appropriate choice of electrode geometry, it is possible to achieve at appropriate regions within the electrode geometry, regions where particular species of particle segregate or particular particle agglomerations occur. In order to enable adequate quantities of suspension to be treated, the electrodes may take the form of a repeating pattern array or, e.g. two electrodes may be comblike and inter-engaged with each extending part of each lying between two neighbouring such parts of the other.

The present invention may be practised using suitable apparatus including a treatment cell including an electrode array, means for feeding a suspension of particles in a liquid to the treatment cell, and means for removing liquid from the cell, first means connected to electrodes in the cell and adapted to generate a first non-uniform electrical field within the cell, and second means connected to electrodes in the cell and adapted to generate a second non-uniform electrical field within the cell and of frequencies differing from the first electrical field.

Preferably, the electrode array is mounted on an external wall of the treatment cell. Such an arrangement may include as part of the liquid removing means perforations in the external wall of the cell bearing the electrodes, the perforations being so located that when the electrodes are appropriately electrically activated relative to the particles in the liquid medium in the cell and to the liquid medium itself, the liquid and particles drawn off through the perforations will differ from the general bulk characteristics of the suspension of particles in the liquid in the cell.

In the description of specific illustrated apparatus which follows, for simplicity of expression, reference is made to applying signals to electrodes. It should be understood that in order to create the appropriate electric fields, the signal is, in practice, applied across a pair of electrodes, one of which may be a reference electrode or an extensive surface area 'ground plate' or the like.

Depending upon the nature of the particles in the suspension, the nature of the suspension itself, and the nature of the applied electrical fields to the electrode array, the particles may be caused to aggregate towards regions of an electrode or a number of electrodes or alternatively detach themselves from such electrode(s) and/or aggregate towards regions away from the electrodes. These phenomena may be exploited in a wide variety of applications to manipulate the particles in suspension, e.g. to provide the directed assembly of structures from such suspended particles, or to provide separation from a mixture of suspended particles of different types, to provide characterization of particular particle types or to promote a reaction between particles of two or more different types.

The accompanying drawings and the examples which follow the description thereof will serve to illustrate the techniques used in practising the method of the invention in more detail. In the drawings:
Figures 1a and 1b show top, side and end views of two different examples of electrode geometries which may be used in the method of the present invention,
Figures 2 and 3 show diagrammatically the movement of a particle using the electrode array of Figure 1a,
Figure 4 shows diagrammatically a more complex situation,
Figures 5a, 5b and 5c show the electrode geometry of Figure 1b and examples of particle aggregation at regions on the electrodes and regions away from the electrodes,
Figure 6 shows diagrammatically two different particle types collected at the same time using both positive and negative dielectrophoretic forces,
Figure 7a shows the random distribution of two particle types in the regions near two independent pairs of electrodes,
Figure 7b shows the resulting distribution of two particle types after the independent electrode pairs have been energized by two characteristically different voltages, and

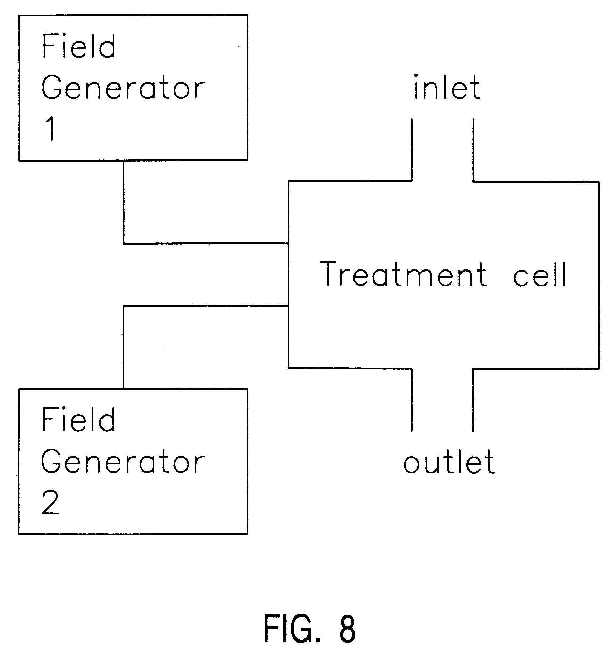

Figure 8 shows a block diagram of apparatus which can be used to carry out the method of the invention.

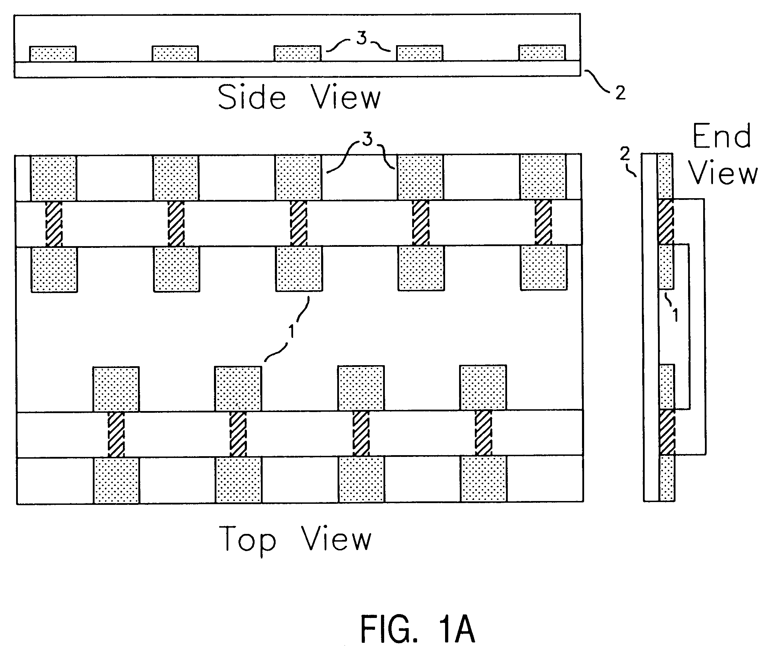

Referring to the drawings, Figure 1a shows in plan, side and end views, one wall of a treatment cell which can be used to manipulate a suspension consisting of one or more particle types. The apparatus comprises an array of electrodes 1 fabricated on to a suitable substrate 2 that forms the wall or surface of the treatment cell. Each electrode can be individually and independently energized by any form of electrical signal via electrical connectors 3. The electrodes may be in direct electrical contact with the particle suspending liquid or separated from it by an appropriate material. For the purpose of illustration the electrodes in Figure 1a are shown having a rectangular geometry, but other geometries may be used depending on the particle characteristics and the desired effect to be achieved.

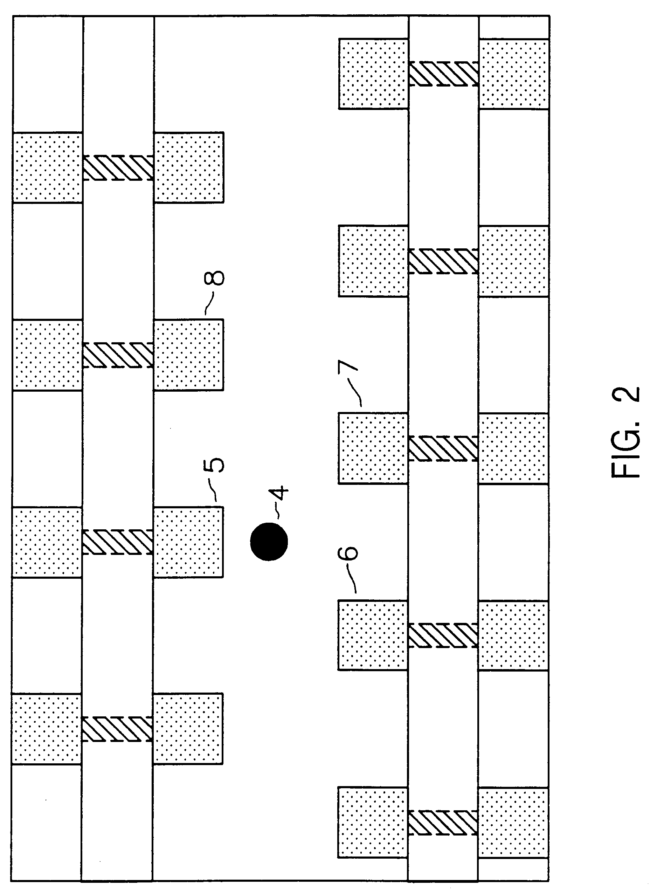

Figure 2 shows the array of electrodes and a test particle 4 suspended in a liquid adjacent to the electrodes. By applying an appropriate electrical signal to electrode 5, the particle can be dielectrophoretically attracted towards electrode 5. This effect is enhanced by applying, at the same time, another electrical signal to electrodes 6 and 7 such that particle 4 is dielectrophoretically repelled from electrodes 6 and 7. The electrical signals are applied to electrodes 5, 6 and 7 until particle 4 becomes immobilized at electrode 5 or reaches a desired locality in the region of electrode 5. The particle can then be further moved by applying, for example, electrical signals which repel the particle from the region of electrode 5 and attract it towards electrode 8.

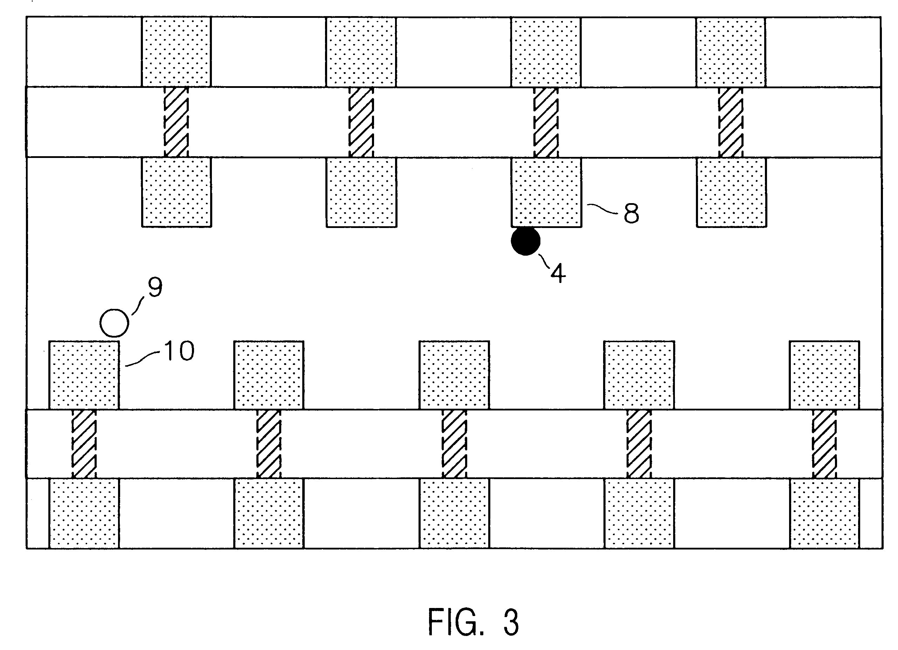

Figure 3 shows particle 4 after it has been dielectrophoretically manipulated to the region of electrode 8 and also shows another particle 9 located at an electrode 10. Particle 9 may or may not have the same dielectric and conductive properties as particle 4. Particles 4 and 9 may be positioned alongside each other by dielectrophoretically moving either or both of them. Bringing particles 4 and 9 (and any others in like fashion) into association may be effected for the purpose of constructing larger building blocks or for inducing a specific chemical, biological or electrochemical reaction between them. This example describes the bringing together of particles but, in the more general case, the apparatus may be employed to manipulate particles into any desired positions relative to each other.

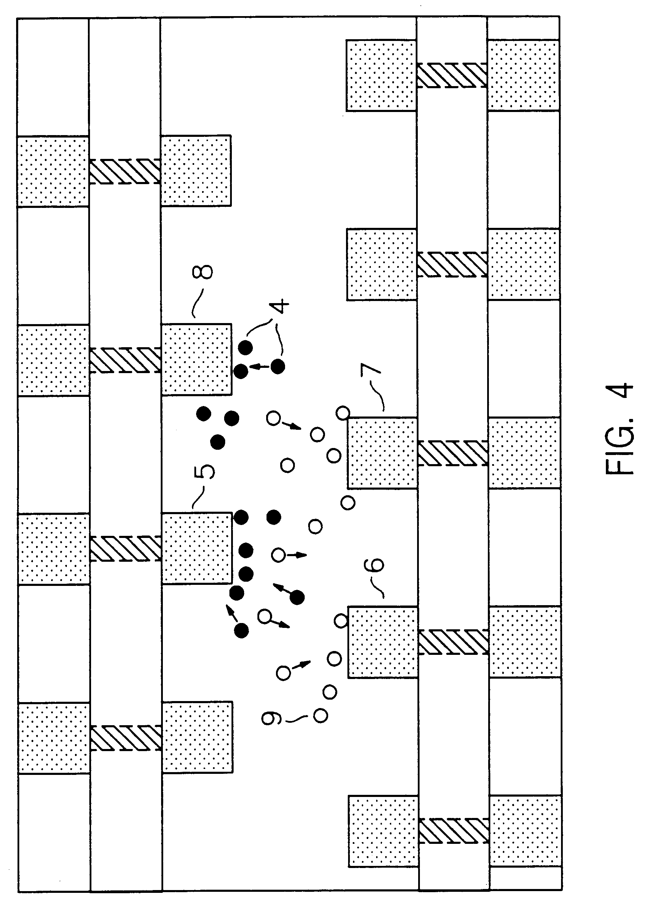

In Figure 4 a collection of particles is shown composed of particle types 4 and 9, which in this case possess differing bulk and/or surface electrical properties. By applying an electrical signal to electrodes 5 and 8 that differs from the signal applied to electrodes 6 and 7, and by an appropriate choice of signal characteristics (i.e. waveform, magnitude and frequency) as well as the suspending medium characteristics (e.g. pH, dielectric constant, conductivity and specific density) particle types 4 and 6 may be physically separated from each other with, for example, particle type 4 collecting near electrodes 5 and 8 and particle type 9 collecting near electrodes 6 and 7. The electrical signals applied to electrode pairs 5 + 8 and 6 + 7, or any other electrode combination, may be applied continuously or intermittently, and at the same or differing times, in order to achieve the desired separation. The particle types may then be removed separately from the treatment cell by drawing off the particle suspending fluid through perforations located near the electrodes, having first released the desired particle type from the electrodes either by removing the electrical signal used to collect them or, if strong electrode adhesion occurs, releasing them by applying electrical signals of appropriate characteristics to the electrodes. The extent of particle collection at the electrodes can be continuously assessed using an optical monitoring technique as described by Burt, Al-Ameen and Pethig in the Journal of Physics, Section E, Scientific Instrumentation, Volume 22 (1989) pages 952 to 957, and the subsequent release of particles can likewise be monitored by an optical probe at the electrodes and also downstream of the perforations.

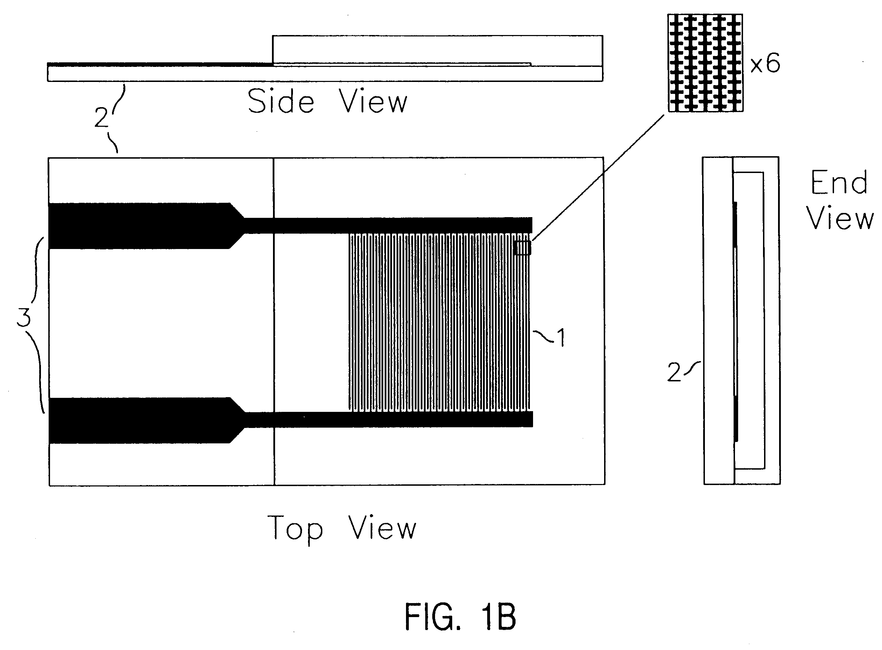

Figure 1b shows, in plan, side and end views, another electrode geometry which can be used to manipulate a suspension consisting of one or more particle types. For the purpose of illustration, the electrodes in Figure 1b are shown having a castellated, interdigitated, rectangular, geometry, but other geometries may be used depending on the particle characteristics and the desired effect to be achieved.

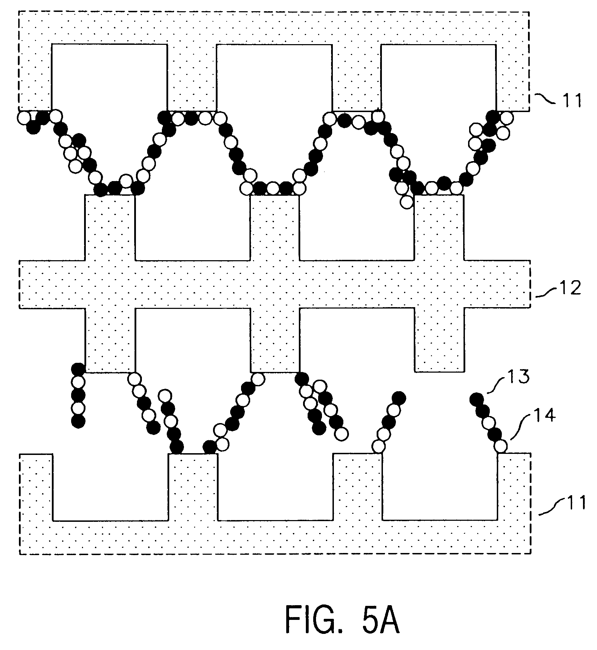

Figure 5a shows diagrammatically a section of an array of interdigitated, castellated, electrodes 11 and 12 after the application of a voltage between electrodes 11 and 12. Two different particle types 13 and 14 have been aggregated as long chains at the outer tips of the individual electrode castellations, as a result of both particle types experiencing a positive dielectrophoretic force.

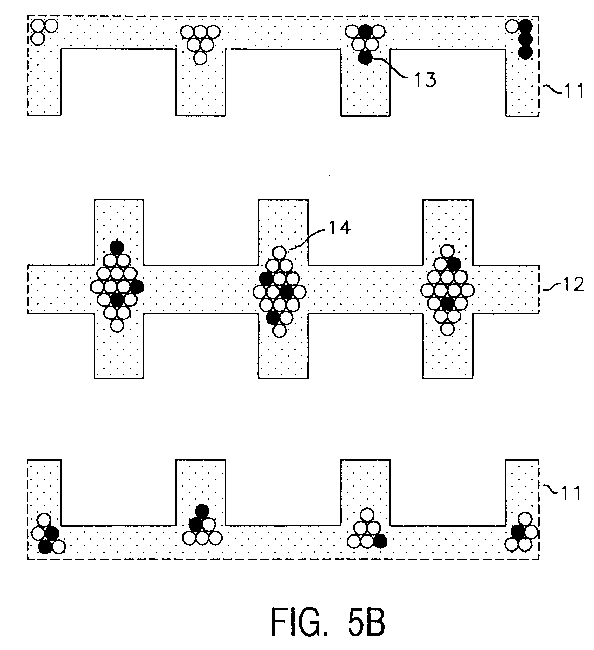

Figure 5b shows diagrammatically the same type of electrode configuration as in Figure 5a, where two different particle types 13 and 14 have been aggregated into regions of the upper electrode surfaces, away from the electrode sides, as a result of experiencing a negative dielectrophoretic force.

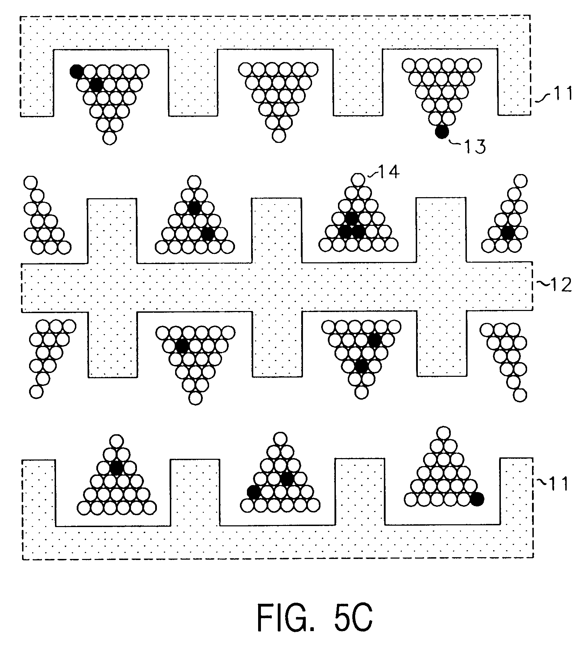

Figure 5c shows diagrammatically the same type of electrode configuration as in Figures 5a and 5b, where two different particle types 13 and 14 have been directed into forming triangular-shaped aggregations in the regions between the electrode castellations away from the electrode sides, as a result of experiencing a negative dielectrophoretic force.

### EXAMPLE 1

A cell was taken with an array of interdigitated, castellated, electrodes substantially as shown in Figure 1b. Each castellation was 20 microns wide, 40 microns deep, about 0.1 micron high, spaced at 80 micron centres, the interdigitated electrode rows being 80 microns apart. The entire array had 60 electrodes in each row, and was around 5mm long. The array was located on one wall of a cell of internal volume 7.5 cubic mm.

A suspension in a medium of 280 nM mannitol in deionised water containing as suspended particles Micrococcus lysodeikticus (ellipsoids around 2 microns long and 0.5 micron across) was added in equal amount to a suspension of latex particles (1.27 micron diameter) in deionised water. The concentration of suspended particles was such that the optical absorbence at a wavelength of 635 nm, and 1 cm path length was, in each case, 1.61 (cf deionised water). The conductivity of the Micrococcus suspension was 11.4 micro-Siemens per cm, whilst that of the latex particle suspension was 2.1 micro-Siemens per cm. When evenly distributed, the amount of interaction between the latex particles and the Micrococcus was very small.

On application of a voltage of 4V p/p sinewave at a frequency of 100 kHz, the latex and Micrococcus particles aggregated as long chains at the outer tips of the individual electrode castellations in a similar manner to that shown in Figure 3 of the 1988 Biochimica et Biophysica Acta paper of Price, Burt and Pethig, and as also schematically shown in Figure 5a. The term "positive dielectrophoresis" as used herein is to be interpreted broadly as this form of particle aggregation in which the particles move towards the areas of higher field strength. On removal of the applied voltage, the latex and Micrococcus particles separated from each other and became dispersed in the suspending medium. This process of bringing the latex and Micrococcus particles into intimate contact with each other, and then letting them separate, can be repeated many times.

On application of a voltage of 4V p/p sinewave at a frequency in the range between 100 Hz and 1 kHz, the latex and Micrococcus particles aggregated at regions of the upper electrode surfaces, away from the electrode sides, as illustrated in Figure 5b. This form of particle aggregation, where the particles are directed away from high electric field regions at electrode edges, is not the normal form of positive dielectrophoresis, and herein is to be interpreted broadly as negative dielectrophoresis.

### EXAMPLE 2

A cell was taken of the same form as that in Example 1, but each electrode castellation was 80 microns wide, 80 microns deep, about 0.1 micron high, spaced at 160 micron centres and with the interdigitated electrode rows being spaced at 160 microns apart. The entire electrode array had 60 electrodes in each row, and was around 1.0 cm long. The array was located on one wall of a cell of internal volume 30 cubic mm.

A suspension in a medium of 280 mM mannitol in deionised water was prepared containing as suspended particles equal numbers of live Brewers yeast cells and dead (autoclaved) Brewers yeast cells to an optical absorbance of about 0.8 at a wavelength of 635 nm for 1cm path length. On application of a voltage of 20V p/p sinewave at a frequency range of 100 Hz to 20 MHz, both the live and dead yeast cells experienced a positive dielectrophoretic force and collected at the outer tips of the electrode castellations.

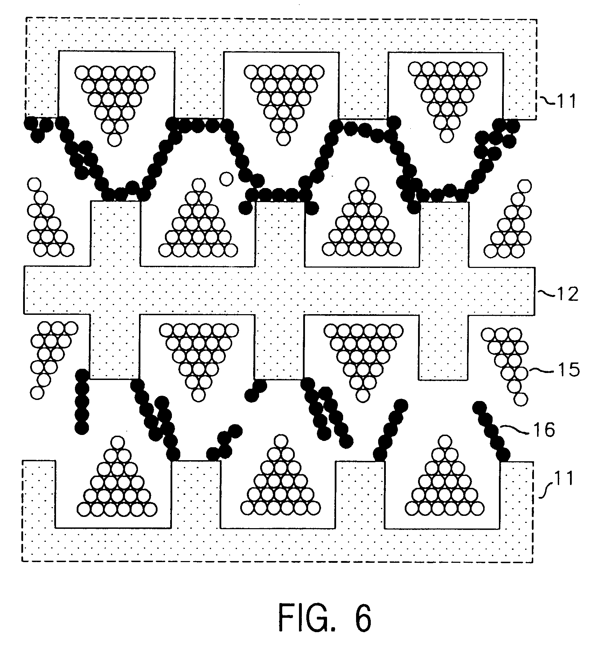

On increasing the electrical conductivity of the mannitol suspending medium, by the addition of potassium chloride, to a conductivity of 150 micro-Siemens per cm, then, depending on the frequency of the voltage applied to the electrodes, the two cell types could each be made to experience either a negative or positive dielectrophoretic force. For example, when a voltage of 20V p/p sinewave at a frequency of 10 kHz was applied to the electrodes, the dead yeast cells were observed to collect at the outer tips of the electrode castellations (as shown in Figure 5a) as a result of experiencing a positive dielectrophoretic force, whilst the live yeast cells were directed by a negative dielectrophoretic force into a triangular-shaped aggregation in the regions between the electrode castellations away from the electrode sides, as shown in Figure 5c. The overall collection of the live and dead yeast cells is similar to that shown in Figure 6, where the live yeast cells are labelled as particle type 15 and the dead yeast cells are labelled as particle type 16. On the other hand, if a voltage of 20V p/p sinewave at a frequency of 10 MHz was applied to the electrodes, then the live yeast cells experienced a positive dielectrophoretic force and collected in the form of Figure 5a, whilst the dead cells experienced a negative dielectrophoretic force and aggregated in the triangular form as shown in Figure 5b.

### EXAMPLE 3

A cell was taken with an array of interdigitated, castellated, electrodes of the same geometry and dimensions as that used in Example 1 above. A suspension in a medium of deionized water was prepared, containing as suspended particles two types of latex particles of diameter 1.27 micron. The first type of latex particle was coated with the antibody raised in rabbit against horseradish peroxidase, whilst the second type of latex particle was coated with horseradish peroxidase-labelled antibody raised in swine against the horseradish peroxidase antibody raised in rabbit. The final suspension of electrical conductivity 4.1 micro-Siemens per cm was formed by mixing together in equal volumes a suspension of latex particle type 1, of optical density 0.8 at a wavelength of 635 nm at a 1 cm path length, with a suspension of latex particle type 2 of optical density 0.54 at a wavelength of 635 nm at a 1 cm path length.

On applying to the electrodes a voltage of 4V p/p sinewave at a frequency of 1 kHz, both latex particle types experienced a negative dielectrophoretic force and rapidly aggregated at regions of the upper electrode surfaces, away from the electrode sides, as shown in Figure 5b. This aggregation brought both types of latex particle into close contact with each other, and greatly accelerated the rate of interaction between the two antibody types coated on the latex particles. On removing the applied voltage to the electrodes, a significant number of the latex particles were observed to be bound together as a result of the dielectrophoretically-induced interaction of the latex particles.

### EXAMPLE 4

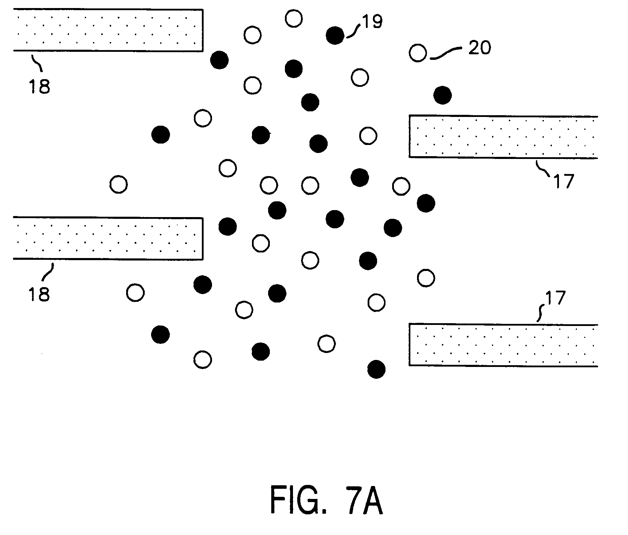

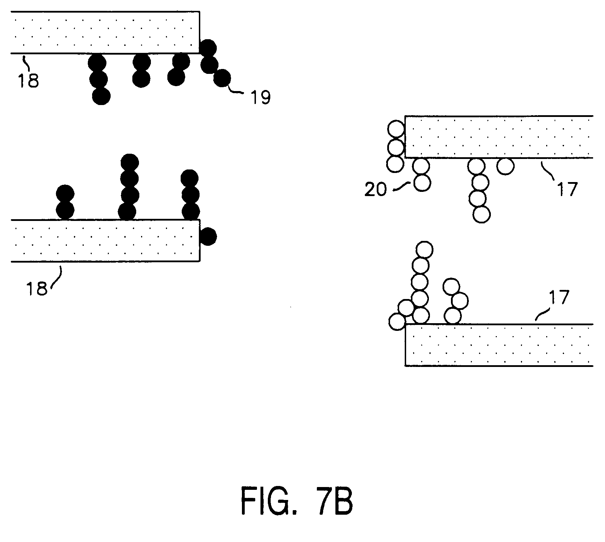

A cell was taken with an electrode geometry similar to that shown in Figures 7a and 7b, where the electrode pair 17 can be energized by an applied voltage separately and independently from electrode pair 18. The separation between the two electrodes forming electrode pair 17 was 104 microns, and likewise for the two electrodes forming electrode pair 18. Each electrode element was of width 32 micron, and of height around 0.1 micron, and the two electrode pairs were spaced 130 microns apart from each other.

A suspension in a medium of 280 mM mannitol in deionized water was prepared, which contained, as suspended particles, equal numbers of live Brewers yeast cells 19 and dead (autoclaved) Brewers yeast cells 20 to an optical absorbance of about 0.8 at a wavelength of 635 nm and a 1 cm optical path length. To this suspension medium was added potassium chloride of sufficient concentration to increase the electrical conductivity of the medium to 150 micro-Siemens per cm. A voltage of 20V p/p sinewave at a frequency of 10 kHz was applied to electrodes 17 of Figures 7a and 7b, and at the same time a voltage of 20V p/p sinewave at a frequency of 10 MHz was applied to electrodes 18. The live yeast cells were observed to be attracted by a positive dielectrophoretic force towards, and to collect at, the electrodes 18 energised by the 10 MHz voltage, and to be repelled by a negative dielectrophoretic force from the regions around the electrodes 17 energized by the 10 kHz voltage. The dead yeast cells, on the other hand, were repelled by a negative dielectrophoretic force from the regions around the electrode 18 energized by the 10 MHz voltage, but were attracted by a positive dielectrophoretic force towards, and collected at, the electrodes 17 energized by the 10 kHz voltage. The distribution of live and dead yeast cells before and after application of the 10 kHz and 10 MHz voltages is shown in Figures 7a and 7b, respectively. As can be seen, a spatial separation of the live yeast cells from the dead yeast cells is accomplished by this procedure.

### EXAMPLE 5

A cell was taken with an array of interdigitated, castellated, electrodes of the same geometry and dimensions as that used in Example 1 above. Two types of glass beads of nominal diameter 1.0 micron were used. The first type of glass bead was shaken in 5% aminopropyl triethoxysilane in dry acetone for 3 hours, then washed and dried at 70 degrees Centigrade. After drying, the beads were shaken in a 5% solution of nitrophenyl ester of d-biotin in chloroform. This procedure resulted in the first type of glass bead being coated with a film of d-biotin. The second type of glass bead was shaken in a solution containing 1mg per ml of avidin in phosphate buffered saline at pH 7.7 for 20 minutes. The treated beads were then washed three times in phosphate buffered saline solution. This procedure resulted in the second type of glass bead being coated with a film of avidin. Beads type 1 and 2 were then separately suspended in a solution of potassium chloride of electrical conductivity 3.5 micro-Siemens per cm.

The final suspension was formed by mixing together in equal volumes a suspension of glass bead type 1, of optical density 0.8 at a wavelength of 635 nm at 1cm optical path length, with a suspension of glass bead type 2 of optical density 0.8 at a wavelength of 635 nm at a 1 cm optical path length.

On applying to the electrodes a voltage of 6V p/p sinewave at a frequency of 800 Hz, both glass particle types experienced a negative dielectrophoretic force and aggregated at regions of the upper electrode surfaces, away from the electrode sides, similar to that shown in Figure 5b. On removal of the applied voltage, a significant number of the glass beads were observed to be firmly bound together as a result of avidin-biotin complexes being formed between the surfaces of glass beads type 1 and 2.

## Claims

1. A method of promoting reactions between particles (4, 9) suspended in a liquid, by using the phenomenon of dielectrophoresis, the particles being of one type or of more than one type, which method comprises introducing a volume of the liquid with particles suspended therein into a treatment cell provided with electrodes, applying an alternating electric signal of relatively high frequency to the electrodes to generate redistribution of the particles under the effect of dielectrophoretic force characterised in that, while the particles in the said cell occupy the redistribution positions, a second alternating electric signal is applied to the electrodes, the frequency of the second signal being significantly different from that of the first signal and chosen taking into account the dielectric constant and electrical conductivity of the particles and the liquid to produce dielectrophoretic forces acting on the particles differing from those produced by the first signal, and effecting a second redistribution of the particles differing from the first redistribution.

2. A method of promoting reactions between particles (4, 9) suspended in a liquid, by using the phenomenon of dielectrophoresis, the particles being of one type or of more than one type, which method comprises introducing a volume of the liquid with particles suspended therein into a treatment cell provided with electrodes, applying an alternating electric signal of relatively high frequency to the electrodes to generate redistribution of the particles under the effect of dielectrophoretic force characterized in that simultaneously a second alternating electric signal is applied to the electrodes, the frequency of the second signal being significantly different from that of the first signal and chosen taking into account the dielectric constant and electrical conductivity of the particles and the liquid to produce dielectrophoretic forces acting on the particles differing from those produced by the first signal.

3. A method according to Claim 1 or 2, characterized in that at least one of the alternating electric signals applies a negative dielectrophoretic force on some only of the particles in the liquid.

4. A method according to any one of Claims 1 to 3 and including the step of modifying the electrical conductivity and/or the dielectric constants of the liquid and/or the particles.

## Patentansprüche

1. Verfahren zum Fördern von Reaktionen zwischen Partikeln (4, 9), die in einer Flüssigkeit in Suspension gehalten sind, durch Verwendung des Phänomens der Dielektrophorese, wobei die Partikel von einem Typ oder von mehr als einem Typ sind, wobei das Verfahren ein Einführen eines Volumens der Flüssigkeit mit den darin in Suspension gehaltenen Partikeln in eine Behandlungszelle, die mit Elektroden versehen ist, und ein Anlegen eines alternierenden elektrischen Signals relativ hoher Frequenz an die Elektroden zum Erzeugen einer Neuverteilung der Partikel unter der Wirkung dielektrophoretischer Kraft aufweist, dadurch gekennzeichnet, daß, während die Partikel in der Zelle die Neuverteilungsstellen einnehmen, ein zweites alternierendes elektrisches Signal an die Elektroden angelegt wird, wobei sich die Frequenz des zweiten Signals signifikant von derjenigen des ersten Signals unterscheidet und gewählt ist unter Einbeziehung der dielektrischen Konstante und elektrischen Leitfähigkeit der Partikel und der Flüssigkeit, um auf die Partikel wirkende dielektrische Kräfte zu erzeugen, die sich von denjenigen unterscheiden, die von dem ersten Signal erzeugt werden, und um eine zweite Neuverteilung der Partikel zu bewirken, die sich von der ersten Neuverteilung unterscheidet.

2. Verfahren zum Fördern von Reaktionen zwischen Partikeln (4, 9), die in einer Flüssigkeit in Suspension gehalten sind, unter Verwendung des Phänomens der Dielektrophorese, wobei die Partikel von einem Typ oder mehr als einem Typ sind, das ein Einführen eines Volumens der Flüssigkeit mit darin in Suspension gehaltenen Partikeln in eine mit Elektroden versehene Behandlungszelle und ein Anlegen eines alternierenden elektrischen Signals relativ hoher Frequenz an die Elektroden zum Erzeugen einer Neuverteilung der Partikel unter der Wirkung dielektrophoretischer Kraft aufweist, dadurch gekennzeichnet, daß gleichzeitig ein zweites alternierendes elektrisches Signal an die Elektroden angelegt wird, wobei sich die Frequenz des zweiten Signals signifikant von derjenigen des ersten Signals unterscheidet und ausgewählt ist unter Einbeziehung der dielektrischen Konstante und elektrischen Leitfähigkeit der Partikel und der Flüssigkeit, um auf die Partikel wirkende dielektrophoretische Kräfte zu erzeugen, die sich von denjenigen unterscheiden, die durch das erste Signal erzeugt werden.

3. Verfahren nach Anspruch 1 oder 2, dadurch gekennzeichnet, daß zumindest eines der alternierenden elektrischen Signale eine negative dielektrophoretische Kraft auf lediglich einige der Partikel in der Flüssigkeit aufbringt.

4. Verfahren nach einen der Ansprüche 1 bis 3 und einschließlich des Schritts des Modifizierens der elektrischen Leitfähigkeit und/oder der dielektrischen Konstanten der Flüssigkeit und/oder der Partikel.

## Revendications

1. Procédé pour favoriser des réactions entre des particules (4,9) en suspension dans un liquide, moyennant l'utilisation du phénomène de diélectrophorèse, les particules étant d'un type ou de plus d'un type, le procédé consistant à introduire un volume du liquide dans lequel des particules sont en suspension, dans une cellule de traitement équipée d'électrodes, appliquer un signal électrique alternatif de fréquence relativement élevée aux électrodes pour produire une redistribution des particules sous l'effet de la force diélectrophorétique, caractérisé en ce que, alors que les particules situées dans ladite cellule occupent les positions de redistribution, un second signal électrique alternatif est appliqué aux électrodes, la fréquence du second signal étant nettement différente de celle du premier signal et étant choisie en tenant compte de la constante diélectrique et de la conductivité électrique des particules et du liquide pour produire des forces diélectrophorétiques agissant sur les particules différentes de celles produites par le premier signal, et exécuter une seconde redistribution des particules, qui diffère de la première redistribution.

2. Procédé pour favoriser des réactions entre des particules (4,9) en suspension dans un liquide, moyennant l'utilisation du phénomène de diélectrophorèse, les particules étant d'un type ou de plus d'un type, le procédé consistant à introduire un volume du liquide dans lequel des particules sont en suspension, dans une cellule de traitement équipée d'électrodes, appliquer un signal électrique alternatif de fréquence relativement élevée aux électrodes pour produire une redistribution des particules sous l'effet de la force diélectrophorétique, caractérisé en ce que simultanément un second signal électrique alternatif est appliqué aux électrodes, la fréquence du second signal étant nettement différente de celle du premier signal et étant choisie en tenant compte de la constante diélectrique et de la conductivité électrique des particules et du liquide pour produire des forces diélectrophorétiques agissant sur les particules, qui diffèrent de celles produites par le premier signal.

3. Procédé selon la revendication 1 ou 2, caractérisé en ce qu'au moins l'un des signaux électriques alternatifs applique une force diélectrophorétique négative uniquement à certaines des particules dans le liquide.

4. Procédé selon l'une quelconque des revendications 1 à 3 et incluant l'étape consistant à modifier la conductivité électrique et/ou les constantes diélectriques du liquide et/ou des particules.
